Europäisches Patentamt

European Patent Office

Office européen des brevets

(11) Veröffentlichungsnummer : **0 376 917 B1**

# (12) EUROPÄISCHE PATENTSCHRIFT

(45) Veröffentlichungstag der Patentschrift :
**04.08.93 Patentblatt 93/31**

(51) Int. Cl.$^5$ : **A61K 47/10,** A61K 31/44,
A61K 9/12

(21) Anmeldenummer : **89890334.9**

(22) Anmeldetag : **28.12.89**

(54) **Pharmazeutische Zubereitung.**

(30) Priorität : **30.12.88 AT 3211/88**

(43) Veröffentlichungstag der Anmeldung :
**04.07.90 Patentblatt 90/27**

(45) Bekanntmachung des Hinweises auf die
Patenterteilung :
**04.08.93 Patentblatt 93/31**

(84) Benannte Vertragsstaaten :
**BE CH DE ES FR GB GR IT LI LU NL SE**

(56) Entgegenhaltungen :
**EP-A- 0 131 228**
**EP-A- 0 175 671**
**EP-A- 0 240 484**
**EP-A- 0 303 490**
**FR-A- 2 369 867**

(73) Patentinhaber : **Burghart, Kurt, Dr.**
**Sägeberg 8**
**W-2217 Rosdorf (DE)**
Patentinhaber : **Burghart, Walter**
**Salmgasse 4**
**A-1030 Wien (AT)**

(72) Erfinder : **Burghart, Kurt, Dr.**
**Sägeberg 8**
**W-2217 Rosdorf (DE)**
Erfinder : **Burghart, Walter**
**Salmgasse 4**
**A-1030 Wien (AT)**

(74) Vertreter : **Haffner, Thomas M., Dr. et al**
**Patentanwaltskanzlei Dipl.-Ing. Adolf**
**Kretschmer Dr. Thomas M. Haffner**
**Schottengasse 3a**
**A-1014 Wien (AT)**

EP 0 376 917 B1

## Beschreibung

Die Erfindung bezieht sich auf eine pharmazeutische Zubereitung enthaltend Nifedipin als Wirkstoff mit einem Lösungsvermittler, wie z.B. Copolyvidon oder Polyvinylpyrrolidon und/oder Glycerin-Polyethylenglykoloxystearat und einem Lösungsmittel, wie z.B. Alkohole, insbesonders Ethanol, oder Polyalkohole sowie ggf. Treibmittel zum Versprühen als einzeldosierbarer Spray für sublinguale Darreichung.

Nifedipinhaltige Arzneimittel sind in verschiedenster Zusammensetzung für unterschiedliche Darreichungsformen bekannt. Eine Zusammensetzung der eingangs genannten Art ist beispielsweise der WO 87/05211 zu entnehmen. Bei dieser vorbekannten pharmazeutischen Zubereitung wurde die Zubereitung dahingehend verbessert, daß Nifedipin in versprühbarer Form, d.h. mit einem Treibmittel vermischt, in Lösung verbleibt und nach dem Sprühstoß nicht ohne weiteres wiederum ausfällt. Es ist bekannt, daß Nifedipin schlecht wasserlöslich ist, und es ist weiters bekannt, daß eine Zubereitung, in welcher Nifedipin gelöst vorliegt, mit dem Mundspeichel Fällungsreaktionen des Nifedipins ergibt. In diesem Zusammenhang wurde in der "Deutschen Apothekerzeitung", 128. Jahrgang, Nr. 23, auf Seite 38, festgehalten, daß die verschiedenen bekannten oralen Darreichungsformen gelegentlich auch in Packungsbeilagen als sublingual wirksam beschrieben werden, daß diese Wirksamkeit aber nicht verifiziert werden konnte. Für die akute Therapie der hypertensiven Krise und der Angina pectoris vorgeschlagene nicht retardierte Nifedipinkapseln für sublinguale Anwendung sollten gemäß derartigen Packungsbeilagen nach dem Zerbeißen der Kapsel und nach dem Verbleib des Kapselinhaltes für einige Zeit im Mund zu einer rascheren Resorption führen, welche sich in der Praxis nicht beweisen ließ. Dieser Umstand wird darauf zurückgeführt, daß die bekannten Zubereitungen Fällungsreaktionen unterliegen, und daß ein relativ rasches Kristallwachstum des Nifedipins die gewünschte Resorption verhindert.

Für die Beschleunigung der Resorption von Pharmazeutika wurde insbesondere für dermatologische Anwendungen bereits eine Reihe von Hilfsstoffen bekannt, und es wurde im besonderen Benzylalkohol als Resorptionsbeschleuniger vorgeschlagen. Eine resorptionsbeschleunigende Wirkung ist beispielsweise in der EP-A 183 527 im Zusammenhang mit Calcitonin beschrieben worden. Bei der aus dieser EP-A 183 527 bekanntgewordenen Zusammensetzung handelt es sich um eine über die Nasenschleimhäute resorbierbare Zubereitung.

Aus der DE-OS 2 209 526 sind Nifedipin enthaltende Coronarmittel bekanntgeworden, von welchen behauptet wird, daß sie rasch perlingual, also über die Zunge und über die Schleimhaut des Rachens, resorbierbar seien. Kontrollversuche haben nun ergeben, daß die aus der DE-OS 2 209 526 sich zum einen selbst dann nicht für die Herstellung eines Sprays, wenn Treibmittel verwendet werden, eignen und zum andern mit dem Speichel der Mundhöhle ein rasches Kristallwachstum des Nifedipins und eine Fällung ergeben.

Die Bedingungen für eine Rezeptur, welche gleichzeitig mit Treibmittel mischbar sein soll, Nifedipin in Lösung halten soll und Fällungsreaktionen bei Verdünnung mit Wasser bzw. dem Mundspeichel sicher verhindern soll, sind teilweise kontradiktorisch, und insbesondere in jüngster Zeit wird eine höhere Anflutungsgeschwindigkeit bei sublingualer Applikation von Nifedipin enthaltenden Zubereitungen gemäß dem Stand der Technik ernsthaft bezweifelt. Ein derartiger Zweifel ist beispielsweise auch der "Deutschen Apothekerzeitung", 128. Jahrgang, Nr. 24, Seite 1268, zu entnehmen, wo ausdrücklich darauf hingewiesen wird, daß sublinguale Applikationen der bekannten Präparate niedrigere Plasmakonzentrationen im Vergleich zu peroraler Einnahme ergeben. Bei Rezepturen, welche ohne Treibgas mittels einer Pumpe dosierbar sprühbar sein sollen, besteht ein weiteres Problem darin, daß die Viskosität und Oberflächenspannung der Zubereitung nur in engen Grenzen wählbar ist, um eine entsprechende Teilchengröße und Verteilung des Sprühnebels im Mund zu gewährleisten. Auch in diesen Fällen muß Nifedipin in Lösung gehalten werden und es dürfen Fällungsreaktionen bei Verdünnung mit Wasser bzw. dem Mundspeichel keinesfalls auftreten. Bei bisher bekannten Zubereitungen war zum einen die erforderliche Viskosität für einen Sprühstoß nicht erzielt und zum andern sind die bekannten Zubereitungen, für welche eine sublinguale Absorption behauptet wurde, nach der Mitteilung der WHO Drug Information Vol.2, Nr.3, 1988, ineffektiv. In diesem Zusammenhang wird von der WHO auf eine Studie in den Niederlanden verwiesen, bei welcher die sublinguale Resorption von Nifedipin als nicht nachweisbar bezeichnet wurde. Mit der aus der EP-A-0 175 671 bekanntgewordenen Zubereitung ist es erstmals gelungen, eine pharmazeutische Zubereitung mit Nifedipin als Wirkstoff als Aerosol unter Verwendung eines Treibgases oder auch als Pumpenspray für die sublinguale Anwendung zu formulieren.

Die resorptionsbeschleunigende Wirkung von Benzylalkohol konnte bei einer Reihe von pharmazeutisch wirksamen Substanzen nicht nachgewiesen werden, so daß Benzylalkohol gleichfalls nicht generell als für pharmazeutische Wirkstoffe geeignetes Resorptionsbeschleunigungsmittel angesprochen werden kann.

Mit der aus der WO 87/05211 bekanntgewordenen pharmazeutischen Zubereitung ist es gegenüber dem zu diesem Zeitpunkt bekannten Stand der Technik möglich geworden, eine raschere Anflutung und eine um etwa 30% größere Fläche unter der Peakkurve zu erzielen.

EP 0 376 917 B1

Die vorliegende Erfindung zielt nun darauf ab, eine pharmazeutische Zubereitung der eingangs genannten Art zu schaffen, welche sich für das Versprühen mit oder ohne Treibmittel für sublinguale Anwendung eignet und eine noch raschere Anflutung und insbesondere eine höhere Plasmakonzentration in kurzer Zeit nach der Darreichung sowie über einen längeren Zeitraum ergibt. Die Zubereitung soll hiebei einzeldosierbar sein, um therapeutischen Ansprüchen zu genügen. Gleichzeitig soll hiebei eine Steigerung der sublingualen Resorption erreicht werden. Zur Lösung dieser Aufgabe besteht die pharmazeutische Zubereitung der eingangs genannten Art im wesentlichen darin, daß die sprühbare Zubereitung 0,5-30 Gew.-% Benzylalkohol enthält. Die lokalanästhetische Wirkung von Benzylalkohol ist bekannt. Die Dosierung darf aus diesem Grunde 30 Gew.-% keinesfalls überschreiten. Die Verwendung von Benzylalkohol hat nun überraschenderweise ergeben, daß Benzylalkohol nicht nur als hervorragendes Lösungsmittel für Nifedipin anzusprechen ist, sondern daß die Zugabe von Benzylalkohol bei gleichzeitiger Anwesenheit von Copolyvidon und Glycerin-Polyethylenglykoloxystearat zum einen die Fällung des Nifedipins beim Auftreffen auf den Mundspeichel mit Sicherheit hintanhält und gleichzeitig die perlinguale bzw. sublinguale Resorption wesentlich beschleunigt. Innerhalb des erfindungsgemäß angegebenen Bereiches konnten Steigerungen der resorbierten Menge an Nifedipin um 60 bis 70% gegenüber den bekannten Zubereitungen für perorale Darreichung und immer noch eine beachtliche Steigerung von etwa 30% der Resorption gegenüber der aus der WO 87/05211 bekannten pharmazeutischen Zubereitung beobachtet werden, welche keinen Benzylalkohol enthielt.

Die Substanz "Copolyvidon" ist ein Copolymer aus Polyvinylpyrrolidon und Vinylacetat.

In besonders vorteilhafter Weise ist bei der Verwendung von Treibmittel Benzylalkohol in Mengen bis zu 15 Gew.-%, vorzugsweise bis zu 10 Gew.-% bezogen auf die Lösung abzüglich Treibmittel, eingesetzt, um auf diese Weise unerwünschte Nebenwirkungen, insbesondere ein starkes Brennen in der Mundhöhle und ein Überwiegen des lokalanästhetischen Effektes, zu vermeiden.

Als besonders vorteilhaft hat sich herausgestellt, daß die Zubereitung in einem Sprühstoß von 75 bis 250 mg 1-7,5 Gew.-% Nifedipin, 20-40 Gew.-% Glycerin-Polyethylenglykoloxystearat, 1-5 Gew.-% Benzylalkohol, 15-25 Gew.-% Ethanol, 0,5-5 Gew.-% Copolyvidon und 30-45 Gew.-% Treibgas sowie ggf. pharmazeutisch übliche Süß- und Geschmacksstoffe in Mengen bis zu 2,5 Gew.-% enthält. Mit Zubereitungen, welche den Wirkstoff sowie den Resorptionsbeschleuniger und Lösungsmittel bzw. Lösungsvermittler in den angegebenen Prozentsätzen enthalten, wird eine besonders schnelle Anflutung und eine hohe Plasmakonzentration innerhalb kurzer Zeit erzielt.

Eine besonders vorteilhafte Zubereitung im Rahmen der vorliegenden Erfindung ist im wesentlichen dadurch gekennzeichnet, daß die Zubereitung 5 mg Nifedipin, 40-60 mg Glycerin-Polyethylenglykoloxystearat, 25-40 mg Ethanol, 1-10 mg Copolyvidon, 1-15 mg Benzylalkohol und 50-70 mg Treibgas sowie ggf. pharmazeutisch übliche Süß- und Geschmacksstoffe in einem Sprühstoß von 125 bis 200 mg enthält.

Bei Verwendung von unter Pumpendruck versprühbaren Zubereitungen haben sich pharmazeutische Zubereitungen, welche in einem Sprühstoß von 50 bis 300 mg 1-5 Gew.-% Nifedipin, 20-50 Gew.-% Glycerin-Polyethylenglykoloxystearat, 2-12 Gew.-% Benzylalkohol, bis 5 Gew.-% Copolyvidon, 25-60 Gew.-% Ethanol und/oder Polyethylenglykol und 0,5-35 Gew.-% Wasser sowie ggf. übliche Süß- und Geschmacksstoffe in Mengen bis zu 2,5 Gew.-% enthalten, als besonders vorteilhaft herausgestellt. Auch mit derartigen Zubereitungen werden eine besondere schnelle Anflutung und hohe Plasmaspiegel des Wirkstoffes innerhalb eines kurzen Zeitraumes nach der Verabreichung erreicht.

Als besonders vorteilhaft hat sich im Rahmen der vorliegenden Erfindung eine unter Pumpendruck versprühbare Zubereitung, welche 1-5 mg Nifedipin, 35-65 mg Glycerin-Polyethylenglykoloxystearat, 1-15 mg Benzylalkohol, 2-8 mg Copolyvidon, 30-45 mg Ethanol und 1-15 mg Wasser sowie gegebenenfalls pharmazeutisch übliche Süß- und Geschmacksstoffe bei einem Sprühstoß von 70 bis 150 mg enthält, herausgestellt.

Kontrollversuche, wie sie insbesondere vom Bundesgesundheitsamt ausdrücklich gefordert werden, haben gezeigt, daß relevante Aussagen für die Wirkungsweise ausschließlich aus Messungen des Blutplasmaspiegels des Wirkstoffes gewonnen werden können. Die in der Literatur vereinzelt zu findenden Rückspülversuche, bei welchen in die Mundhöhle gesprühte Pharmazeutika wiederum eluiert werden, um auf diese Weise einen Differenzwert zum resorbierten Anteil zu erhalten, scheitern im vorliegenden Fall daran, daß bei einer Fällung des Wirkstoffes der gefällte Anteil beim Rückspülversuch gleichfalls nicht ausgespült wird bzw. in der nachfolgenden Messung nicht korrekt erfaßt wird. Der Zusatz von Benzylalkohol im Rahmen der erfindungsgemäßen Zubereitung hat nicht nur die Verbesserung der Resorption des Pharmazeutikums erbracht, sondern auch eine Vorverlegung des wirksamen Zeitpunktes, zu welchem pharmazeutisch wirksame Blutplasmakonzentrationen vorliegen.

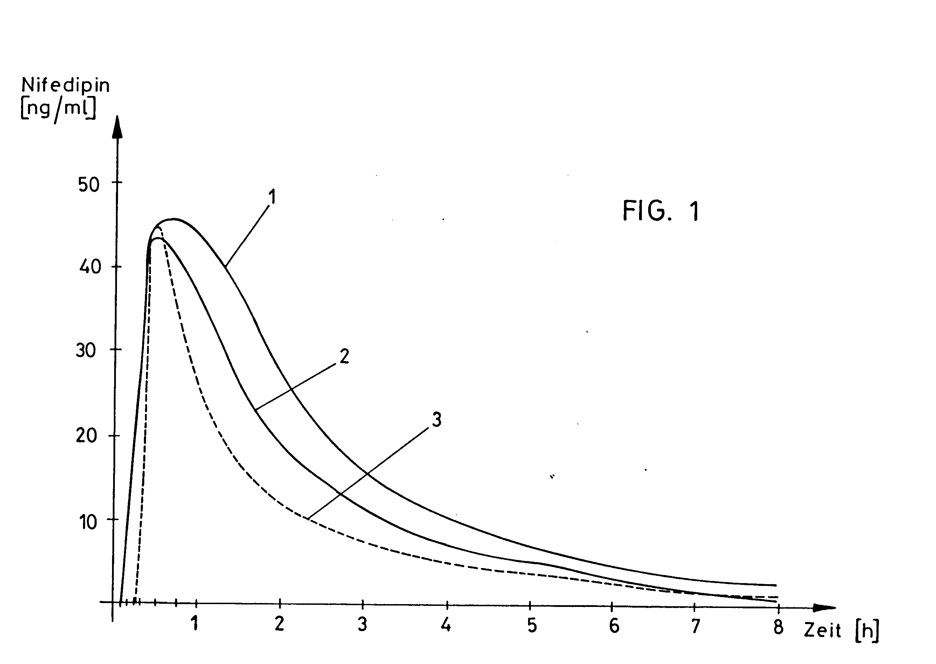

Die wesentlichen Vorteile der erfindungsgemäßen Zubereitungen gegenüber bekannten Zubereitungen ergeben sich aus den in der Zeichnung und in dem Beispiel dargestellten Vergleichsversuchen. In der Zeichnung zeigt Fig. 1 die Mittelwerte der Pharmaspiegel aus drei Probandenversuchen zur Ermittlung der relativen Bioverfügbarkeit für eine erfindungsgemäße Nifedipin enthaltende Zubereitung und zwei Nifedipin enthalten-

3

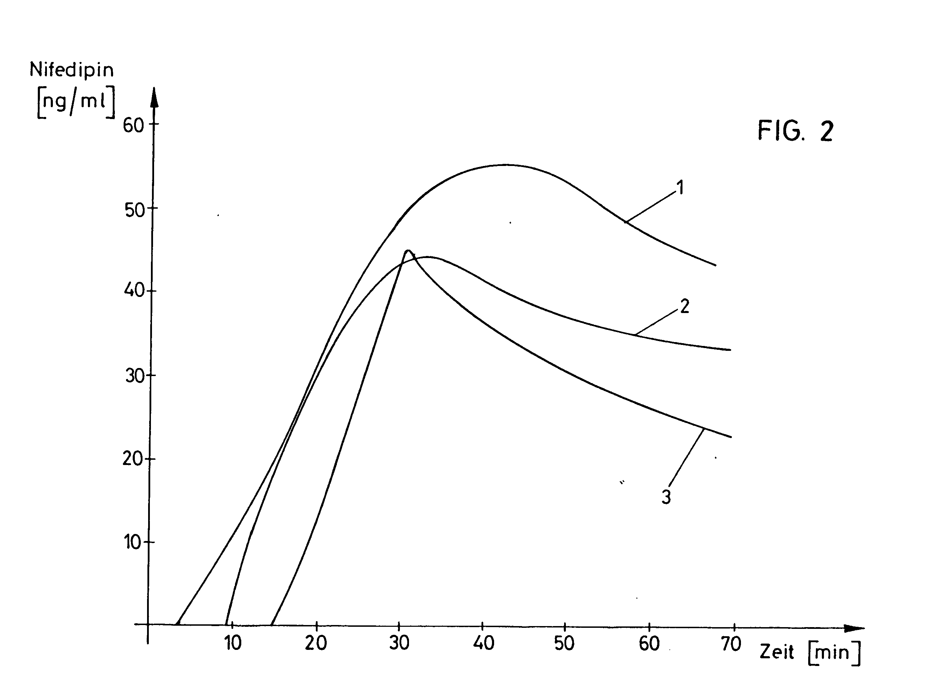

de Zubereitungen nach dem Stand der Technik innerhalb von 8 h. Fig. 2 zeigt die Mittelwerte aus drei Probandenversuchen des Anflutungsprofiles der relativen Bioverfügbarkeit für eine erfindungsgemäße Nifedipin enthaltende Zubereitung und zwei Zubereitungen nach dem Stand der Technik, welche ebenfalls Nifedipin enthalten, innerhalb der ersten Stunde nach der Verabreichung. Die gemessenen Werte sind hiebei jeweils in Nanogramm pro Milliliter auf der Ordinate gegen die Zeit in Stunden bzw. Minuten auf der Abszisse dargestellt.

Als Vergleichsproben wurde in der Fig. 1 einerseits eine handelsübliche Zerbeißkapsel, welche 5 mg Nifedipin pro Kapsel enthält, herangezogen und andererseits eine sprühfähige Zubereitung nach dem Stand der Technik, welche 5 mg Nifedipin, 50 mg Glycerin-Polyethylenglykoloxystearat, 30 mg Ethanol, 5 mg Copolyvidon und 60 mg Treibgas enthält, herangezogen. Die in der Fig. 1 eingesetzte erfindungsgemäße Zubereitung enthält in insgesamt 160 mg Treibgasspray, 5 mg Nifedipin, 50 mg Glycerin-Polyethylenglykoloxystearat, 30 mg Ethanol, 5 mg Copolyvidon, 10 mg Benzylalkohol und 60 mg eines aus Fluorchlorkohlenwasserstoffen bestehenden Treibgases, sowie ggf. übliche Süß- und Geschmacksstoffe.

In der Fig. 1 ist die Kurve, welche sich durch die Anwendung der erfindungsgemäßen sprühfähigen Zubereitung ergab, mit 1 bezeichnet. Diejenige, welche die sprühfähige nifedipinhaltige Zubereitung nach dem Stand der Technik enthielt, mit 2 bezeichnet und diejenige, welche für eine Zerbeißkapsel nach dem Stand der Technik erhalten wurde, mit 3 bezeichnet. Die Vergleichswerte, welche für die Zerbeißkapsel erhalten wurden, ergaben sich nach einer oralen Verabreichung, d.h. nach einer Verabreichung, bei welcher die Kapsel unzerkaut geschluckt wurde. Die sprühfähigen Zubereitungen 1 und 2 wurden jeweils sublingual angewandt. Aus dem Vergleichsversuch ergibt sich, daß bei den beiden sprühfähigen Zubereitungen bereits nach 8 min die ersten Serumwerte an Nifedipin im Blut gemessen werden konnten. Bei der in Kapselform verabreichten Zubereitung konnte ein erster Serumwert von 1,7 ng Nifedipin pro Milliliter erst nach 15 min nachgewiesen werden, zu welchem Zeitpunkt die beiden sprühfähigen Zubereitungen bereits Serumwerte von etwa 20 ng pro Milliliter Nifedipin im Plasma aufwiesen. Nach einem Zeitraum von 30 min bis zu einem Zeitraum von 45 min nach der Verabreichung erreichen die Plasmaspiegel sowohl von der kapselförmigen Zubereitung als auch von den beiden sprühförmigen Mischungen ihre maximalen Plasmakonzentrationen. Hiebei liegen die maximalen Plasmakonzentrationen der sprühfähigen Mischung nach dem Stand der Technik bei 44 ng pro Milliliter Nifedipin im Plasma und diejenigen der kapselförmigen Zubereitung und der erfindungsgemäßen sprühfähigen Mischung bei jeweils 46 ng pro Milliliter Nifedipin im Plasma. Nach 40 min beginnen die Plasmaspiegel von sowohl der kapselförmigen Zubereitung als auch der sprühfähigen Zubereitung nach dem Stand der Technik bereits stark abzufallen, wohingegen die erfindungsgemäße sprühfähige Zubereitung ihren maximalen Plasmawert erst nach 50 min mit 47 ng pro Milliliter Nifedipin im Plasma erreicht. Nach diesem Zeitpunkt fällt auch der Plasmaspiegel der erfindungsgemäßen Zubereitung ab, jedoch ist der Abfall gegenüber den Zubereitungen nach dem Stand der Technik bedeutend weniger steil, und die Plasmakonzentration bleibt bis zu einem Zeitpunkt von 8 h deutlich über denjenigen Konzentrationen, welche die beiden Zubereitungen nach dem Stand der Technik aufweisen. Die mittleren Konzentrationen von Nifedipin im Plasma ist in der nachfolgenden Tabelle aufgelistet.

## Tabelle 1

### Nifedipin im Plasma
(ng/ml)

| Zeit | Kapsel | Spray Std.d.T. | erf.Zuber. |
|---|---|---|---|
| 10 min | -- | 4,1 | 4,5 |
| 15 min | 0,6 | 19,7 | 20,2 |
| 30 min | 45,2 | 43,6 | 45,5 |
| 45 min | 36,6 | 38,7 | 47,0 |
| 60 min | 26,2 | 36,6 | 41,2 |
| 90 min | 15,9 | 25,8 | 37,8 |
| 2 h | 11,8 | 18,4 | 25,7 |
| 3 h | 8,4 | 11,8 | 16,2 |
| 4 h | 4,9 | 7,3 | 10,6 |
| 5 h | 3,8 | 5,0 | 7,2 |
| 6 h | 2,9 | 3,4 | 4,9 |
| 7 h | 1,5 | 2,4 | 3,8 |
| 8 h | 0,6 | 1,4 | 3,0 |

Aus dieser Figur sowie der oben gezeigten Tabelle ergibt sich zweifelsfrei, daß bei der erfindungsgemäßen Zubereitung die höchsten Konzentrationen von Nifedipin im Plasma erreicht werden können. Die Fläche unter der Kurve, welche ein Maß für die Menge an wirksamem Arzneistoff im Körper darstellt, ist bei der erfindungsgemäßen Zubereitung um 72% größer als bei der kapselförmigen Zubereitung und um 30% größer als bei der versprühbaren Zubereitung nach dem Stand der Technik.

Bei einer Reihe von in der Zeichnung nicht dargestellten Vergleichsversuchen wurden andere Substanzen erprobt, für welche eine Verbesserung der Resorption bereits in der Literatur behauptet wurde. Überraschenderweise hat sich hiebei gezeigt, daß die gleichfalls bekannten Resorptionsbeschleuniger, Ethylacetat oder Benzoesäure, im Zusammenhang mit Nifedipin keine wie immer geartete Wirkung bzw. Verbesserung des Resorptionsverhaltens ergeben haben.

Als Vergleichsproben wurde in der Fig. 2 einerseits eine handelsübliche Zerbeißkapsel, welche 5 mg Nifedipin pro Kapsel enthält, herangezogen und andererseits eine sprühfähige Zubereitung nach dem Stand der Technik, welche 5 mg Nifedipin, 50 mg Glycerin-Polyethylenglykoloxystearat, 30 mg Ethanol, 5 mg Copolyvidon und 60 mg Treibgas enthält, herangezogen. Die in der Fig. 2 eingesetzte erfindungsgemäße Zubereitung enthält in insgesamt 160 mg Treibgasspray, 5 mg Nifedipin, 50 mg Glycerin-Polyethylenglykoloxystearat, 30 mg Ethanol, 5 mg Copolyvidon, 5 mg Benzylalkohol und 65 mg eines aus Fluorchlorkohlenwasserstoffen bestehenden Treibgases, sowie ggf. übliche Süß- und Geschmacksstoffe.

In dieser Figur ist wiederum die Kurve, welche sich durch die Anwendung der erfindungsgemäßen Zubereitung ergab, mit 1 bezeichnet. Diejenige, welche die sprühfähige Zubereitung nach dem Stand der Technik enthielt, mit 2 bezeichnet und diejenige, welche für eine nifedipinhaltige Zerbeißkapsel erhalten wurde, mit 3 bezeichnet. Die Vergleichswerte für die Zerbeißkapsel ergaben sich nach einer oralen Anwendung der Kapsel, bei welcher diese unzerkaut geschluckt wurde. Die sprühfähigen Zubereitungen wurden jeweils sublingual verabreicht. Aus dem Vergleichsversuch ergibt sich, daß bei der erfindungsgemäßen sprühfähigen Zubereitung bereits nach 4 min ein erster Serumwert von 1,13 ng pro Milliliter Nifedipin im Plasma gemessen werden kann. Ein erster Serumwert bei dem Spray nach dem Stand der Technik konnte nach 10 min gemessen werden und dieser betrug 4,1 ng pro Milliliter Nifedipin im Plasma. Zu diesem Zeitpunkt betrug der Plasmaspiegel der erfindungsgemäßen sprühfähigen benzylalkohol- und nifedipinhaltigen Zubereitung bereits 11,7 ng pro Milliliter Nifedipin im Plasma. Für die kapselförmige Zubereitung konnte ein erster Serumwert erst nach 15 min bestimmt werden und dieser betrug 0,6 ng pro Milliliter Nifedipin im Plasma. Nach 30 min erreichen sowohl die Kapsel als auch der Spray nach dem Stand der Technik ihre höchsten Plasmaspiegel, nämlich im Falle der Kapsel 45,3 ng pro Milliliter Nifedipin im Plasma und im Falle des Sprays nach dem Stand der Technik 43,7 ng pro Milliliter Nifedipin im Plasma. Zu diesem Zeitpunkt wird für die erfindungsgemäße Zubereitung ein Serumwert von 51,2 ng pro Milliliter Nifedipin im Plasma gemessen. Die erfindungsgemäße sprühfähige Zubereitung erreicht ihren höchsten Plasmawert erst nach 45 min mit 56,0 ng pro Milliliter Nifedipin im Plasma. Danach beginnt auch der Plasmaspiegel der erfindungsgemäßen Zubereitung abzufallen, jedoch ist dieser Abfall nicht so steil wie beispielsweise bei der kapselförmigen Zubereitung nach dem Stand der Technik. In der nachfolgenden Tabelle ist das Anflutungsprofil von Nifedipin im Plasma gezeigt.

## Tabelle 2

Anflutungsprofil von Nifedipin im Plasma (ng/ml)

| Zeit (min) | Kapsel | Spray Std.d.T. | erfgem. Zuber. |
|---|---|---|---|
| 2 | 0 | 0 | 0 |
| 4 | 0 | 0 | 1,13 |
| 6 | 0 | 0 | 4,7 |
| 8 | 0 | 0 | 8,16 |
| 10 | 0 | 4,1 | 11,7 |
| 15 | 0,6 | 19,6 | 16,5 |
| 20 | 12,8 | 30,9 | 31,1 |
| 30 | 45,3 | 43,7 | 51,2 |
| 45 | 36,0 | 38,8 | 56,0 |
| 60 | 26,2 | 36,5 | 46,9 |

Aus Fig. 2 sowie der oben gezeigten Tabelle zeigt sich deutlich, daß mit der erfindungsgemäßen sprühfähigen Zubereitung eine bedeutend raschere Anflutung von Nifedipin im Plasma erzielt werden kann. Weiters ist aus Tabelle 2 sowie Fig. 2 deutlich ersichtlich, daß nicht nur eine raschere Anflutung mit der erfindungsgemäßen Zubereitung erzielt werden kann, sondern auch eine höhere Plasmakonzentration in kurzer Zeit gegenüber den zwei Zubereitungen nach dem Stand der Technik erzielt werden kann. So liegt die maximale Plasmakonzentration ($C_{max.}$) der erfindungsgemäßen sprühfähigen Zubereitung mit 56,0 ng pro Milliliter Nifedipin im Plasma deutlich höher als diejenigen der beiden Zubereitungen nach dem Stand der Technik, welche jeweils im Falle der Kapsel eine maximale Plasmakonzentration von 45,3 ng pro Milliliter Nifedipin im Plasma und im Falle der sprühfähigen Zubereitung von 43,7 ng pro Milliliter Nifedipin im Plasma aufweisen.

Beispiel 1:

Fünf Nifedipin enthaltende Zubereitungen werden in einem Versuch, bei welchem die Verhältnisse bei sublingualer Applikation simuliert werden, dahingehend untersucht, wie lange die Zubereitungen unter diesen Bedingungen stabil bleiben, d.h. wie lange keine Auskristallisation von Nifedipin auftritt.

|  | Vergl. | Zus. 1 | Zus. 2 | Zus. 3 | Zus. 4 |
|---|---|---|---|---|---|
| Nifedipin | 5 mg | 5 mg | 5 mg | 5 mg | 5 mg |
| Glycerin-<br>-Polyethylen-<br>glykoloxy-<br>stearat | 50 mg | 50 mg | 50 mg | 50 mg | 50 mg |
| Ethanol | 30 mg | 35 mg | 30 mg | 30 mg | 30 mg |
| Copolyvidon | 5 mg | 5 mg | 5 mg | 5 mg | 5 mg |
| Benzyl-<br>alkohol | -- | 2 mg | 5 mg | 10 mg | 13 mg |
| Treibgas | 60 mg | 63 mg | 65 mg | 60 mg | 57 mg |
|  | 150 mg | 160 mg | 160 mg | 160 mg | 160 mg |

In einem Uhrglas werden jeweils 0,1 ml Wasser bzw. künstlicher Speichel vorgelegt, ein Sprühstoß von 160 mg bzw. 150 mg, im Falle der Zubereitung nach dem Stand der Technik, abgegeben und nach einer weiteren Minute werden weitere 0,5 ml Wasser bzw. künstlicher Speichel zugesetzt. Bei der Verbindung nach dem Stand der Technik wurde nach 15 min kein Auskristallisieren der Nifedipinkristalle beobachtet und bei sämtlichen erfindungsgemäßen Zubereitungen lag die Dauer bis zum Auftreten der ersten Nifedipinkristalle im gleichen Zeitraum, obwohl Benzylalkohol zugesetzt wurde. Demgegenüber wird, wenn eine Zubereitung für Kapseln nach dem Stand der Technik, wie beispielsweise die in Beispiel 4 der DE-OS 22 09 526 angeführte Zubereitung, diesem Versuch unterworfen wird, sofort nach der Zugabe von 0,5 ml Wasser bzw. künstlichem Speichel ein Auskristallisieren beobachtet.

Mit den oben gezeigten Zubereitungen wurden weiters Penetrationsversuche durchgeführt. Bei diesen Penetrationsversuchen wurde jeweils die Penetration an Nifedipin nach einem Zeitraum von 3 min bestimmt.

Bei der sprühfähigen Zubereitung nach dem Stand der Technik wurde nach dem angegebenen Zeitraum eine Resorption von 0,13 mg Nifedipin errechnet. Die erfindungsgemäßen Zubereitungen, welche ansteigende Mengen an Benzylalkohol enthielten, zeigten jeweils eine größere Resorption an Nifedipin. So wurde bei der Zubereitung 1, welche 2 mg Benzylalkohol pro 160 mg Spray enthält, eine Resorption von 0,142 mg errechnet. Bei der Zubereitung 2, welche 5 mg Benzylalkohol enthält, eine Resorption von 0,156 mg errechnet. Bei der Zubereitung 3, welche 10 mg Benzylalkohol pro 160 mg Spray enthält, errechnete sich die Resorption mit 0,185 mg. Schließlich wurde die Resorption für die Zubereitung 4, in welcher 13 mg Benzylalkohol enthalten waren, mit 0,192 mg errechnet.

Beispiel 2:

Zwei Nifedipin enthaltende Zubereitungen, welche kein Treibmittel enthalten, wurden in bzeug auf ihr Sprühverhalten sowie auf ihre Stabilität unter sublinguale Applikation simulierenden Bedingungen, d.h. wie lange keine Auskristallisation von Nifedipin auftritt, untersucht.

|  | Zus. 1 | Zus. 2 |
|---|---|---|
| Nifedipin | 5,0 mg | 3,85 mg |
| Glycerin-Polyethylen-glykoloxystearat | 57,6 mg | 44,3 mg |
| Benzylalkohol | 13,0 mg | 10,0 mg |
| Copolyvidon | 5,0 mg | 3,85 mg |
| Ethanol | 39,0 mg | 34,0 mg |
| Wasser | 10,4 mg | 4,0 mg |
|  | 130 mg | 100 mg |

In einem Uhrglas werden jeweils 0,1 ml Wasser vorgelegt, ein Sprühstoß von 130 mg bzw. 100 mg abgegeben und nach weiteren 2 min werden weitere 0,5 ml Wasser zugesetzt. Beide Zubereitungen bleiben unter diesen Bedingungen über einen Zeitraum von mindestens 15 min stabil und es wurde kein Auskristallisieren des Nifedipins beobachtet.

Beide Zubereitungen wurden außerdem in ein lichtgeschütztes mit einer Valois-Dosierpumpe versehenes Behältnis gefüllt und die Dosierungsgenauigkeit des Ventils mit dem jeweiligen Spray sowie die Homogenität der Teilchen überprüft.

Im Falle der Zusammensetzung 1 sollten Sprühstoße mit einem Gewicht von 130 mg erzielt werden. Bei der Abgabe von 10 Sprühstößen wurde ein mittleres Dosiergewicht von 130,2 mg erreicht. Der Sprühstoß mit dem höchsten Sprühgewicht hatte hiebei 134,7 mg, derjenige mit dem niedrigsten Gewicht 124,8 mg. Die Sprühstöße wiesen durchwegs eine homogene Teilchengröße auf.

Im Falle der Zusammensetzung 2 sollte ein Gewicht von 100 mg/Sprühstoß erzielt werden. Bei der Abgabe von 10 Sprühstößen wurde ein mittleres Dosiergewicht von 102,1 mg erzielt. Der Sprühstoß mit dem höchsten Sprühgewicht hatte hiebei 105,5 mg, derjenige mit dem niedrigsten Gewicht 96,4 mg.

Beispiel 3:

Drei Nifedipin enthaltende Zubereitungen, welche kein Treibmittel enthalten, wurden in bezug auf ihr Sprühverhalten untersucht.

|  | Zus. 1 | Zus. 2 | Zus. 3 |
|---|---|---|---|
| Nifedipin | 5,0 | 4,0 | 5,0 |
| Glycerin-Polyethylen-glykoloxystearat | 62,6 | 65,0 | 70,0 |
| Benzylalkohol | 13,0 | 14,0 | 14,0 |
| Ethanol | 39,0 | 45,0 | 41,0 |
| Wasser | 10,4 | 17,0 | 10,0 |
|  | 130,0 mg | 140,0 mg | 140,0 mg |

Um einen besonders fein- und gleichmäßig versprühbaren Spray zu erhalten, wurde den drei Zusammensetzungen als Lösungsvermittler jeweils nur Glycerin-Polyethylenglykoloxystearat zugesetzt. Die Zubereitungen wurden in ein lichtgeschütztes mit einer Valois-Dosierpumpe versehenes Behältnis gefüllt und die Dosierungsgenauigkeit des Ventils mit dem jeweiligen Spray sowie die Homogenität der Teilchen überprüft.

Im Falle der Zusammensetzung 1 sollten Sprühstoße mit einem Gewicht von 130 mg erzielt werden. Bei der Abgabe von 10 Sprühstößen wurde ein mittleres Dosiergewicht von 130,4 mg erreicht. Der Sprühstoß mit dem höchsten Sprühgewicht hatte hiebei 135,2 mg, derjenige mit dem niedrigsten Gewicht 126,5 mg. Die Sprühstöße wiesen durchwegs eine homogene Teilchengröße und eine gleichmäßige feine Verteilung der Aerosoltröpfchen auf.

Im Falle der Zusammensetzung 2 sollte ein Gewicht von 140 mg/Sprühstoß erzielt werden. Bei der Abgabe von 10 Sprühstößen wurde ein mittleres Dosiergewicht von 142,1 mg erzielt. Der Sprühstoß mit dem höchsten Sprühgewicht hatte hiebei 143,9 mg, derjenige mit dem niedrigsten Gewicht 138,6 mg.

Im Falle der Zusammensetzung 3 sollte ein Gewicht von 140 mg/Sprühstoß erzielt werden. Nach Abgabe von 10 Sprühstößen wurde ein mittleres Dosiergewicht von 140,9 mg/Sprühstoß erzielt. Der Sprühstoß mit dem höchsten Sprühgewicht hatte hiebei 143,1 mg und jener mit dem niedrigsten Gewicht 137,2 mg.

Alle drei Zusammensetzungen zeichneten sich durch eine besonders leichte Versprühbarkeit und sehr geringe Schwankungen des Sprühgewichtes aus.

Beispiel 4:

Vier Nifedipin enthaltende Pumpenspray-Zubereitungen wurden sowohl in bezug auf ihr Sprühverhalten als auch in bezug auf ihre Stabilität unter einer sublinguale Applikation simulierenden Bedingung untersucht.

|  | Zus. 1 | Zus. 2 | Zus. 3 | Zus. 4 |
|---|---|---|---|---|
| Nifedipin | 5,0 | 5,0 | 6,0 | 5,0 |
| Glycerin-Polyethylen-glykoloxystearat | 75,0 | 60,0 | 90,0 | 75,0 |
| Benzylalkohol | 15,0 | 15,0 | 10,0 | 15,0 |
| Ethanol | 70,0 | 50,0 | 160,0 | 104,0 |
| Polyethylen-glykol 400 | -- | 40,0 | -- | 70,0 |
| Wasser | 34,0 | 79,0 | 30,0 | 30,0 |
| Copolyvidon | 1,0 | 1,0 | 4,0 | 1,0 |
| | 200,0 mg | 250,0 mg | 300,0 mg | 300,0 mg |

In einem Uhrglas werden jeweils 0,1 ml Wasser vorgelegt, ein Sprühstoß von 250 mg bzw. 300 mg abgegeben und nach weiteren 2 min werden weitere 0,5 ml Wasser zugesetzt. Alle Zubereitungen bleiben unter diesen Bedingungen über einen Zeitraum von mindestens 15 min stabil und es wurde kein Auskristallisieren des Nifedipins beobachtet.

Die Zubereitungen wurden in ein lichtgeschütztes mit einer Valois-Dosierpumpe versehenes Behältnis gefüllt und die Dosierungsgenauigkeit des Ventils mit dem jeweiligen Spray sowie die Homogenität der Teilchen überprüft.

Im Falle der Zusammensetzung 1 sollten Sprühstoße mit einem Gewicht von 200 mg erzielt werden. Bei der Abgabe von 10 Sprühstößen wurde ein mittleres Dosiergewicht von 200,9 mg erreicht. Der Sprühstoß mit dem höchsten Sprühgewicht hatte hiebei 203,1 mg, derjenige mit dem niedrigsten Gewicht 196,1 mg. Die Sprühstöße wiesen durchwegs eine homogene Teilchengröße auf.

Im Falle der Zusammensetzung 2 sollte ein Gewicht von 250 mg/Sprühstoß erzielt werden. Bei der Abgabe von 10 Sprühstößen wurde ein mittleres Dosiergewicht von 251,7 mg erzielt. Der Sprühstoß mit dem höchsten Sprühgewicht hatte hiebei 254,3 mg, derjenige mit dem niedrigsten Gewicht 247,0 mg.

Mit der Zusammensetzung 3 sollten Sprühstöße mit einem Gewicht von 300 mg erreicht werden. Nach Abgabe von 10 Sprühstoßen wurde ein mittleres Sprühgewicht von 301,3 mg erzielt, wobei der Sprühstoß mit dem geringsten Gewicht 298,4 mg und jener mit dem höchsten Sprühgewicht 303,6 mg aufwies.

Im Falle der Zusammensetzung 4 sollte ein Gewicht von 300 mg/Sprühstoß erzielt werden. Bei der Abgabe von 10 Sprühstößen wurde ein mittleres Dosiergewicht von 300,8 mg erzielt. Der Sprühstoß mit dem höchsten Sprühgewicht hatte hiebei 302,1 mg, derjenige mit dem niedrigsten Gewicht 297,9 mg.

**Patentansprüche**

1. Pharmazeutische Zubereitung enthaltend Nifedipin als Wirkstoff mit einem Lösungsvermittler, wie z.B. Copolyvidon oder Polyvinylpyrrolidon und/oder Glycerin-Polyethylenglykoloxystearat und einem Lösungsmittel, wie z.B. Alkohole, insbesondere Ethanol, oder Polyalkohole sowie ggf. Treibmittel zum Versprühen als einzeldosierbarer Spray für sublinguale Darreichung, dadurch gekennzeichnet, daß die sprühbare Zubereitung 0,5 bis 30 Gew.-% Benzylalkohol enthält.

2. Pharmazeutische Zubereitung nach Anspruch 1, dadurch gekennzeichnet, daß bei Verwendung von Treibmittel Benzylalkohol in Mengen von bis zu 15, vorzugsweise bis zu 10 Gew.-% bezogen auf die Lösung abzüglich Treibmittel eingesetzt ist.

3. Pharmazeutische Zubereitung nach Anspruch 1 oder 2, dadurch gekennzeichnet, daß die Zubereitung in einem Sprühstoß von 75 bis 250 mg 1-7,5 Gew.-% Nifedipin, 20-40 Gew.-% Glycerin-Polyethylenglykoloxystearat, 1-5 Gew.-% Benzylalkohol, 15-25 Gew.-% Ethanol, 0,5-5 Gew.-% Copolyvidon und 30-45 Gew.-% Treibgas sowie ggf. pharmazeutisch übliche Süß- und Geschmacksstoffe in Mengen bis zu 2,5 Gew.-% enthält.

4. Pharmazeutische Zubereitung nach Anspruch 1, 2 oder 3, dadurch gekennzeichnet, daß die Zubereitung 5 mg Nifedipin, 40-60 mg Glycerin-Polyethylenglykoloxystearat, 25-40 mg Ethanol, 1-10 mg Copolyvidon, 1-15 mg Benzylalkohol und 50-70 mg Treibgas, sowie gegebenenfalls pharmazeutisch übliche Süß- und Geschmacksstoffe in einem Sprühstoß von 125 bis 200 mg, enthält.

5. Pharmazeutische Zubereitung nach Anspruch 1 oder 2, dadurch gekennzeichnet, daß die unter Pumpendruck versprühbare Zubereitung in einem Sprühstoß von 50 bis 300 mg 1-5 Gew.-% Nifedipin, 20-50 Gew.-% Glycerin-Polyethylenglykoloxystearat, 2-12 Gew.-% Benzylalkohol, bis 5 Gew.-% Copolyvidon, 25-60 Gew.-% Ethanol und/oder Polyethylenglykol und 0,5-35 Gew.-% Wasser sowie ggf. übliche Süß- und Geschmacksstoffe in Mengen bis zu 2,5 Gew.-% enthält.

6. Pharmazeutische Zubereitung nach Anspruch 1, 2 oder 5, dadurch gekennzeichnet, daß die unter Pumpendruck versprühbare Zubereitung 1-5 mg Nifedipin, 35-65 mg Glycerin-Polyethylenglykoloxystearat, 1-15 mg Benzylalkohol, 2-8 mg Copolyvidon, 30-45 mg Ethanol und 1-15 mg Wasser sowie gegebenenfalls pharmazeutisch übliche Süß- und Geschmacksstoffe in einem Sprühstoß von 70 bis 150 mg enthält.

## Claims

1. Pharmaceutical preparation, comprising nifedipine as the active ingredient, together with a solubiliser such as copolyvidone or polyvinyl pyrrolidone and/or glycerinepolyethylene glycol oxystearate and a solvent, such as alcohols, especially ethanol, or polyalcohols and, if necessary, a propelling agent for spraying as single dosis spray for sublingual administration, characterized in that the sprayable preparation contains 0,5 to 30 % by weight of benzylic alcohol.

2. Pharmaceutical preparation according to claim 1, characterized in that when a propelling agent is used, benzylic alcohol is present in amounts up to 15, especially up to 10 % by weight relative to the total weight of the solution without propelling agent.

3. Pharmaceutical preparation according to claim 1 or 2, characterized in that the preparation contains in a spray jet of 75 to 250 mg 1 to 7,5 % by weight nifedipine, 20 to 40 % by weight glycerine-polyethylene glycoloxy stearate, 1 to 5 % by weight benzylic alcohol; 15 to 25 % by weight ethanol, 0,5 to 5 % by weight copolyvidone and 30 to 45 % by weight propelling agent as well as, if necessary, pharmaceutically usual sweetening and flavouring agents in amounts of up to 2,5 % by weight.

4. Pharmaceutical preparation according to any of the claims 1, 2 or 3, characterized in that the preparation contains in one spray jet 5 mg nifedipine, 40 to 60 mg glycerine-polyethylene glycoloxy stearate, 25 to 40 mg ethanol, 1 to 10 mg copolyvidone, 1 to 15 mg benzylic alcohol, and 50 to 70 mg propelling agent as well as, if necessary, pharmaceutically usual sweetening and flavouring agents.

5. Pharmaceutical preparation according to claim 1 or 2, characterized in that the preparation sprayable under pump pressure contains in one spray jet of 50 to 300 mg 1 to 5 % by weight nifedipine, 20 to 50 % by weight glycerine-polyethylene glycoloxy stearate, 2 to 12 % by weight benzylic alcohol, up to 5 % by weight copolyvidone, 25 to 60 % by weight ethanol and/or polyethylene glycol, and 0,5 to 35 % by weight water as well as, if necessary, pharmaceutically usual sweetening and flavouring agents in amounts up to 2,5 % by weight.

6. Pharmaceutical preparation according to any of the claims 1, 2 or 5, characterized in that the preparation sprayable under pump pressure contains in one spray jet of 70 to 150 mg 1 to 5 mg nifedipine, 35 to 65 mg glycerine-polyethylene glycoloxy stearate, 1 to 15 mg benzylic alcohol, 2 to 8 mg copolyvidone, 30 to 45 mg ethanol and 1 to 15 mg water as well as, if necessary, pharmaceutically usual sweetening and flavouring agents.

**Revendications**

1. Composition pharmaceutique contenant nifédipine comme matière active avec un solubiliseur, comme copolyvidone ou polyvinylpyrrolidone et/ou l'ester polyéthylèneglycoloxyglycérinique de l'acide stéarique et un solvant, comme des alcools, en particulier éthanol, ou des polyalcools ainsi que le cas échéant des propellants pour atomiser comme spray dosable individuel pour application sublingual, characterisée en ce que la préparation atomisable contiens 0,5 à 30 % en poids de l' alcool benzylique.

2. Composition pharmaceutique selon la revendication 1 characterisée en ce que en utilisant d' un propellant l'alcool benzylique est usé dans une quantité jusqu' à 15, de préférance jusqu' à 10 % en poids relative au poids total de la solution diminué par le propellant.

3. Composition pharmaceutique selon une des revendications 1 ou 2, characterisée en ce que la composition contiens dans un volume de 75 à 250 mg de l' aérosol 1 à 7,5 % en poids nifédipine, 20 à 40 % en poids de l'ester polyéthylène-glycoloxy-glycérinique de l'acide stéarique, 1 à 5 % en poids alcool benzylique, 15 à 25 % en poids éthanol, 0,5 à 5 % en poids copolyvidone et 30 à 45 % en poids propellant ainsi que le cas échéant saccharines et aromates usuel en pharmacie dans une quantité jusqu' à 2,5 % en poids.

4. Composition pharmaceutique selon une des revendications 1, 2 ou 3, characterisée en ce que la composition contiens dans un volume de 125 à 200 mg de l' aérosol 5 mg nifédipine, 40 à 60 mg de l'ester polyéthylèneglycoloxyglycérinique de l'acide stéarique, 25 à 40 mg éthanol, 1 à 10 mg copolyvidone, 1 à 15 mg alcool benzylique et 50 à 70 mg propellant ainsi que le cas échéant saccharines et aromates pharmaceutique usuel.

5. Composition pharmaceutique selon une des revendications 1 ou 2, characterisée en ce que la préparation atomisable sous la pression de la pompe contiens dans un volume de 50 à 300 mg de l' aérosol 1 à 5 % en poids nifédipine, 20 à 50 % en poids de l'ester polyéthylèneglycoloxy-glycérinique de l'acide stéarique, 2 à 12 % en poids alcool benzylique, jusqu' à 5 % en poids copolyvidone, 25 à 60 % en poids éthanol et/ou polyéthylene glygole et 0,5 à 35 % en poids eau ainsi que le cas échéant saccharines et aromates usuel en pharmacie dans une quantité jusqu'à 2,5 % en poids.

6. Compositon pharmaceutique selon une des revendications 1,2 ou 5, characterisée en ce que la préparation atomisable sous la pression de la pompe contiens dans un volume de 70 à 150 mg de l' aérosol 1 à 5 mg nifédipine, 35 à 65 mg de l'ester polyéthylèneglycoloxy-glycérinique de l'acide stéarique, 1 à 15 mg alcool benzylique, 2 à 8 mg copolyvidone, 30 à 45 mg éthanol, et 1 à 15 mg eau ainsi que le cas échéant saccharines et aromates usuel en pharmacie.

FIG. 1

EP 0 376 917 B1

FIG. 2

Nifedipin [ng/ml]

Zeit [min]

EP 0 376 917 B1